# EUROPEAN PATENT SPECIFICATION

(11) **EP 2 026 064 B1**
(45) Date of publication and mention of the grant of the patent: **09.10.2019**
(21) Application number: 08161506.4
(22) Date of filing: 30.07.2008
(51) Int. Cl.: G01N 27/404, G01N 33/00

(54) **Usage of nitrogen-containing-gas removing means to avoid failure of an electrochemical oxygen flue-gas anlayzer**
Verwendung von Mitteln zur Entfernung von Stickstoff enthaltenden Gasen zur Vermeidung von Fehlern in elektrochemischen Sauerstoffsensoren für Rauchgase
Utilisation de moyens d'élimination du gaz contenant de l'azote pour éviter la défaillance d'un analyseur électrochimique de gaz de combustion à l'oxygène

(30) Priority: 30.07.2007 GB 0714788
(43) Date of publication of application: 18.02.2009
(73) Proprietor: Alphasense Limited, Great Notley Essex CM77 7AA (GB)
(72) Inventor: Saffell, John Robert, Cambridge, Essex CB22 5AR (GB); Dawson, Darryl Hirst, Braintree, Essex CM7 5SP (GB)
(74) Representative: Hindles Limited

(56) References cited:
- EP-A2- 0 293 230
- WO-A1-02/46740
- GB-A- 2 490 601
- US-A- 4 820 386
- US-A- 4 900 405
- US-A- 5 331 310
- US-B1- 6 399 391

## Description

### Field of the invention

The present invention relates to the field of flue gas analysers for measuring the concentration of oxygen in flue gas.

### Background to the invention

Electrochemical oxygen sensors are in common use at the present time for measuring the concentration of oxygen in gas samples. Electrochemical oxygen sensors are used in flue gas analysers for measuring the concentration of oxygen in flue gas. Flue gas is the combustion product which exits through the flues of industrial or domestic boilers for heating water or generating power which burn fossil fuels, such as oil, gas and coal, or other plant derived fuels, such as wood or other biofuels.

Gas sensors capable of sensing oxygen are disclosed in, for example, US4900405 A and US4820386 A1. US4900405 A discloses a voltammetric microsensor structure comprising a substrate having an active area on a surface thereof; a sensing electrode having a portion thereof on said active area; a counter electrode having a portion thereof on said active area; a reference electrode having a portion thereof on said active area; said sensing electrode, said counter electrode and said reference electrode defining respectively therebetween a sensing-counter gap, a sensing-reference gap and a counter-reference gap; a solid polymer electrolyte bridging said gaps; a barrier covering said solid polymer electrolyte electrolyric medium, said barrier being permeable to an analyte gas, said barrier can include nitrogen-containing-gas removing means, e.g. permanganate coated alumina which serves to remove nitric oxide; and an aqueous reservoir internal of said substrate in flow contact with said electrolyte. US4820386 A discloses a fast response diffusion-type sensor cell for the detection of carbon monoxide and other oxidizable or reducible gases comprising a three-electrode hydrated proton-conducting membrane cell configuration, with all electrodes in intimate contact with the same proton-conducting membrane. The gas inlet of the flue analyzer can include nitrogen-containing-gas removing means, e.g. permanganate coated alumina which serves to remove nitric oxide.

It has been known for some time that electrochemical gas sensors sometimes fail when measuring the concentration of oxygen in flue gas emitted by some boiler installations. This failure usually occurs within a few days to months of installation. Sometimes the failure is irreversible; sometimes the failure is reversible and the sensors recover after 1 to 3 days without exposure to flue gas.

The failure of electrochemical oxygen sensors in some boiler installations has presented a serious problem for the gas sensing industry. The reason for the failures has been a mystery. Tests in which electrochemical oxygen sensors are exposed to the individual chemical species which make up typical flue gas do not reproduce the failures.

The present invention aims to provide flue gas analysers comprising electrochemical oxygen sensors, which are suitable for prolonged use in installations where conventional flue gas analysers comprising electrochemical oxygen sensors fail.

Within this specification and the appended claims, the term nitrogen dioxide includes its dimer dinitrogen tetroxide with which nitrogen dioxide is in equilibrium in the gaseous phase.

### Summary of the invention

According to the present invention there is provided the use of nitrogen-containing-gas-removing means to avoid failure of a mass flow controlled electrochemical oxygen sensor in a flue gas analyser according to any of claims 1 to 11.

We have discovered that, surprisingly, electrochemical oxygen sensors fail in the presence of a combination of both nitrogen dioxide and a sufficiently high concentration of water vapour, typically at least 80% relative humidity. Accordingly, by providing a flue gas analyser including an electrochemical oxygen sensor, nitrogen-containing-gas removing means for removing from received gas one or more gaseous species comprising nitrogen and oxygen which are either nitrogen dioxide, or formed from nitrogen dioxide in the presence of sufficient water vapour, which would otherwise lead to damage of the electrochemical oxygen sensor, we have provided a flue gas analyser comprising an electrochemical oxygen sensor which is suitable for prolonged use in installations where conventional flue gas analysers comprising electrochemical oxygen sensors fail.

The flue gas analyser comprises nitrogen-containing-gas removing means for removing from received gas one or more gaseous species comprising nitrogen and oxygen which are either nitrogen dioxide, or formed from nitrogen dioxide in the presence of sufficient water vapour, which would otherwise lead to damage of the electrochemical oxygen sensor. The nitrogen-containing gas removing means should remove the substantial majority of the gaseous species.

By gaseous species formed from nitrogen dioxide in the presence of sufficient water vapour, which would otherwise lead to damage of the electrochemical oxygen sensor, we refer to gaseous species formed from nitrogen dioxide in the presence of sufficient water vapour (typically at least 80%, and preferably at least 90% relative humidity) in sufficient quantities to damage the electrochemical oxygen sensor if they were not removed which would not be formed from nitrogen monoxide (e.g. in the presence of 1,000ppm nitrogen monoxide at 90% relative humidity) in the presence of the same amount of water vapour in sufficient quantities to damage the electrochemical oxygen sensor if they were not removed. The nitrogen-containing-gas removing means may remove a gaseous species which would not in itself damage the electrochemical oxygen sensor but which is part of a series of chemical reactions leading to the formation of a species which would otherwise damage the electrochemical oxygen sensor.

The gaseous species formed from nitrogen dioxide in the presence of sufficient water vapour, which would otherwise lead to damage of the electrochemical oxygen sensor may be one or more of nitric acid (HNO₃), nitrous acid (HNO₂) and dinitrogen pentoxide (N₂O₅). Accordingly, the nitrogen-containing-gas removing means may be nitric acid removing means for removing nitric acid from received gas. The nitrogen-containing-gas removing means may be nitrous acid removing means for removing nitrous acid from received gas. The nitrogen-containing-gas removing means may be dinitrogen pentoxide removing means for removing nitrogen pentoxide from received gas.

The nitrogen-containing-gas removing means may be means for removing nitrogen dioxide from received gas. The nitrogen-containing-gas removing means may remove nitrogen monoxide from received gas, as well as nitrogen dioxide. Alternatively, the nitrogen-containing-gas removing means for removing from received gas one or more gaseous species comprising nitrogen and oxygen which are either nitrogen dioxide, or formed from nitrogen dioxide in the presence of sufficient water vapour, which would otherwise lead to damage of the electrochemical oxygen sensor, but not remove nitrogen monoxide.

The nitrogen-containing-gas removing means may remove a range of acid gases including nitrogen dioxide and nitrogen monoxide from received gas. The nitrogen-containing-gas removing means may remove a range of acid gases including nitrogen dioxide, nitrogen monoxide and sulfur dioxide from received gas. Although we have shown that the removal of nitrogen monoxide and/or sulfur dioxide is not required, it can be more cost effective in some circumstances to use a nitrogen-containing-gas removing means which removes a range of acid gases from received gas in preference to a nitrogen-containing-gas removing means which removes only a specific gaseous species from received gas.

The nitrogen-containing-gas removing means may be a filter. The filter may be a filter which removes nitrogen dioxide from received gas. The filter may be a filter which removes a plurality of acid gases including nitrogen dioxide and nitrogen monoxide from received gas.

The nitrogen-containing-gas removing means may be a catalyst for converting one or more of nitrogen dioxide, or another gaseous species formed from nitrogen dioxide in the presence of sufficient water vapour, which would otherwise lead to damage of the electrochemical oxygen sensor, to a gaseous species which would not damage the electrochemical sensor.

The nitrogen-containing-gas removing means may comprise a permanganate salt, such as potassium permanganate. The use of nitrogen-containing-gas removing means which, like potassium permanganate, changes visual properties (e.g. colour) when it removes gas provides a visual indicator when the nitrogen-containing-gas removing means is coming towards the end of its operational life.

The nitrogen-containing-gas removing means may comprise a nitrogen-containing-gas removing filter material, such as a sheet of nitrogen dioxide adsorbing activated carbon. The nitrogen-containing gas removing filter material may be configured to provide a tortuous path for gas to diffuse through the filter material. This enables a high surface area of filter material to be provided in a compact volume. For example, the nitrogen-containing-gas removing means may comprise a plurality of sheets of nitrogen dioxide adsorbing activated carbon spaced apart by gas impermeable members configured to provide a tortuous path for received gas to diffuse through the sheets of nitrogen dioxide adsorbing activated carbon.

The electrochemical oxygen sensor comprises a cathode which is operable to reduce oxygen. The electrochemical oxygen sensor comprises a mass flow control member which restricts diffusion of gas to the cathode. The mass flow control member is selected such that the reduction of oxygen at the cathode is mass flow limited. Accordingly, the electrochemical oxygen sensor is a mass flow controlled electrochemical oxygen sensor. One skilled in the art will appreciate that partial pressure electrochemical oxygen sensors are not mass flow controlled electrochemical oxygen sensors.

The mass flow control member is a narrow tube, such as a narrow tube which extends through a block of solid material. The narrow tube typically has a diameter of 100 microns or less. The nitrogen-containing-gas removing means could be gas permeable and function as the mass flow control member.

The mass flow control member is typically made from a plastics material, preferably a thermoplastics material, such as acrylonitrile butadiene styrene (ABS), or polycarbonate.

The nitrogen-containing-gas removing means may be located within the mass flow control member. The nitrogen-containing-gas removing means may be located intermediate the inlet and the mass flow control member. These arrangements can reduce damage to the mass flow control member in the presence of both nitrogen dioxide and a high concentration of water vapour, which is useful in embodiments where the mass flow control member is susceptible to damage in the presence of both nitrogen dioxide and a high concentration of water vapour. By "damage" we include blockage of the mass flow control member. The nitrogen-containing-gas removing means may be gas permeable and function as the mass flow control member.

The nitrogen-containing-gas removing means are typically located intermediate the inlet and the cathode. The nitrogen-containing-gas removing means may be in gaseous communication with a gas pathway which conducts analyte gas from the inlet to the cathode. The inlet may be a surface of the nitrogen-containing-gas removing means.

Typically, a gas space is provided between the mass flow control member and the cathode. The gas space may be arranged to facilitate diffusion of oxygen from the mass flow control member across the whole surface of the cathode. The nitrogen-containing-gas removing means may be located within the gas space. The cathode is typically covered by a gas permeable liquid impermeable barrier, such as a layer of hydrophobic microporous polytetralfluoroethylene (PTFE), which facilitates the conduction of oxygen to the cathode while retaining electrolyte. In this case, the gas space is typically intermediate the mass flow control member and the gas permeable liquid impermeable barrier. The gas permeable liquid impermeable barrier may be susceptible to damage in the presence of both nitrogen dioxide and a sufficiently high concentration of water vapour.

The flue gas analyser may further comprise a nitrogen oxide sensor which is operable to measure the concentration of a specific nitrogen oxide, such as nitrogen monoxide, or nitrogen dioxide, or the concentration of a range of nitrogen oxides (referred to in the art as a NOₓ sensor). Where the flue gas analyser comprises both a nitrogen oxide sensor which is operable to measure nitrogen dioxide and nitrogen-containing-gas removing means, the nitrogen oxide sensor is located intermediate the inlet and the nitrogen-containing-gas removing means or in gaseous communication with the inlet other than through the nitrogen-containing-gas removing means. A nitrogen dioxide sensor which removes the majority of nitrogen dioxide which contacts an inlet of the nitrogen dioxide sensor may function as the nitrogen-containing-gas removing means.

The flue gas analyser is typically operable to measure carbon monoxide concentration as well as oxygen concentration. Typically, the flue gas analyser includes carbon monoxide sensing apparatus, in addition to the oxygen sensing apparatus. Typically, both the carbon monoxide sensing apparatus and the oxygen sensing apparatus are in gaseous communication with the same gas inlet of the flue gas analyser. Preferably, the flue gas analyser calculates carbon dioxide concentration from the dilution of oxygen by carbon dioxide. The flue gas analyser may also measure the concentration of other gases, for example sulfur and nitrogen oxide concentrations.

The electrochemical oxygen sensor is preferably based on the oxidation of anode material. For example, the electrochemical oxygen sensor may comprise a lead anode.

An example extends in a second aspect to a flue in gaseous communication with a flue gas analyser according to a first aspect of the present invention. Preferably, the flue conducts flue gas which, were it not for the presence of the nitrogen-containing-gas removing means, would cause the flue gas analyser to be damaged.

Preferably, the flue conducts flue gas which, were it not for the presence of gas removing means, would reduce the operational lifetime of the flue gas analyser by at least a factor of ten.

The gas within the flue comprises water vapour with a relative humidity of at least 80%, preferably at least 90% and more preferably at least 95%. The gas within the flue comprises nitrogen dioxide at a concentration of at least 5 parts per million (ppm). By at least 5ppm, we mean that for each million gas molecules, at least 5 are nitrogen dioxide. The invention also extends to a condensing boiler including a said flue.

The nitrogen-containing-gas removing means may be integrated with or separate to the electrochemical oxygen sensor.

The invention is directed to the use of a flue gas analyser according to claim 1 to measure the concentration of oxygen in a flue gas which, were it not for the presence of the nitrogen-containing-gas removing means, would cause the flue gas analyser to be damaged.

Preferably, were it not for the presence of the nitrogen-containing-gas removing means, the flue gas would reduce the operational lifetime of the flue gas analyser by at least a factor of ten.

The flue gas typically comprises water vapour with a relative humidity of at least 80%, preferably at least 90% and more preferably at least 95%. The flue gas typically comprises nitrogen dioxide at a concentration of at least 5 ppm.

There is provided a method of measuring the concentration of oxygen in a received flue gas comprising nitrogen dioxide and water vapour using an electrochemical oxygen sensor, comprising providing, intermediate an inlet from which the received flue gas is received and a component of the electrochemical oxygen sensor which can be damaged in the presence of nitrogen dioxide and sufficient water vapour, nitrogen-containing-gas removing means for removing from received gas one or more gaseous species comprising nitrogen and oxygen which are either nitrogen dioxide, or formed from nitrogen dioxide in the presence of sufficient water vapour, which would damage the component of the electrochemical oxygen sensor if they were not removed. The electrochemical oxygen sensor is typically part of a flue gas analyzer. Optional features of the flue gas analyser, electrochemical oxygen sensor, flue gas, nitrogen-containing-gas removing means correspond to those discussed above in relation to the first three aspects.

There is provided a method of developing a flue gas analyser which has an improved operational lifetime in the presence of nitrogen dioxide and water vapour with a relative humidity of at least 80% (preferably at least 90%, or at least 95%), comprising comparing the operational lifetime of a flue gas analyser including nitrogen-containing-gas removing means for removing from received gas one or more gaseous species comprising nitrogen and oxygen which are either nitrogen dioxide, or formed from nitrogen dioxide in the presence of sufficient water vapour, which would otherwise lead to damage of the electrochemical oxygen sensor and a flue gas analyser lacking said nitrogen-containing-gas in a gas sample comprising at least 5ppm of nitrogen dioxide (preferably, at least 100ppm of nitrogen dioxide) and having a relative humidity of at least 80% (preferably at least 90%, or at least 95%).

There is provided a method of developing a flue gas analyser which has an improved operational lifetime in the presence of nitrogen dioxide and a high concentration of water vapour comprising comparing the operational lifetime of a first flue gas analyser and a second flue gas analyser including at least one part which is made from a different material to a corresponding part in the first flue gas analyzer in the presence of gas samples comprising at least 5ppm of nitrogen dioxide (preferably, at least 100ppm of nitrogen dioxide) and having a relative humidity of at least 80% (preferably 90% and more preferably at least 95%).

There is provided a method of monitoring the concentration of oxygen in a flue comprising measuring the relative humidity of gas within the flue and, if the relative humidity exceeds a predetermined amount (e.g. 80%, 90% or 95%) bringing a flue gas analyser according to the first aspect of the present invention into gaseous communication with the gas within the flue to monitor the concentration of oxygen within the flue.

It may be desirable to select a flue gas analyser according to the first aspect of the present invention irrespective of whether nitrogen dioxide is present in flue gas in a concentration in excess of a predetermined amount (e.g. 5ppm) because nitrogen dioxide may only be produced intermittently or may be produced in the future.

There is provided a method of monitoring the concentration of oxygen in a flue comprising measuring the relative humidity of gas within the flue and the concentration of nitrogen dioxide in the gas within the flue and, if the relative humidity exceeds a predetermined amount (e.g. 80%, 90% or 95%) and the concentration of nitrogen dioxide exceeds a predetermined amount, bringing a flue gas analyser according to the first aspect of the present invention into gaseous communication with gas within the flue to monitor the concentration of oxygen within the flue.

### Description of the Drawings

An example embodiment of the present invention will now be illustrated with reference to the following Figures in which:
Figure 1 is a schematic diagram of oxygen sensing apparatus according to the present invention, within a flue gas analyser;
Figure 2 is a graph of the output current with time from eight electrochemical oxygen sensors in atmospheric air, with the addition of 1,000ppm nitrogen dioxide and water vapour to 90% relative humidity;
Figure 3 is a graph of the output current with time from eight electrochemical oxygen sensors in atmospheric air, with the addition of 100ppm nitrogen dioxide and water vapour to 98% relative humidity;
Figure 4 is a graph of the output current with time from eight electrochemical oxygen sensors in atmospheric air, with the addition of 1,000ppm nitric oxide and water vapour to 90% relative humidity;
Figure 5 is a graph of the output current with time from eight electrochemical oxygen sensors in atmospheric air, with the addition of 1,000ppm sulfur dioxide and water vapour to 90% relative humidity;
Figure 6 is a graph of the output current with time from eight electrochemical oxygen sensors exposed to dry atmospheric air including 1,000ppm nitric oxide for 17 hours, followed by atmospheric air including 1,000ppm nitric oxide and water vapour to a relative humidity of 90%;
Figure 7 is a graph of the output current with time from eight oxygen sensors exposed to gas prepared from atmospheric air, with the addition of 1,000ppm nitrogen dioxide and water vapour to 95% relative humidity, which was filtered through a Purafil brand filter; and
Figure 8 illustrates an alternative filter for the oxygen sensing apparatus of Figure 1.

### Detailed Description of an Example Embodiment

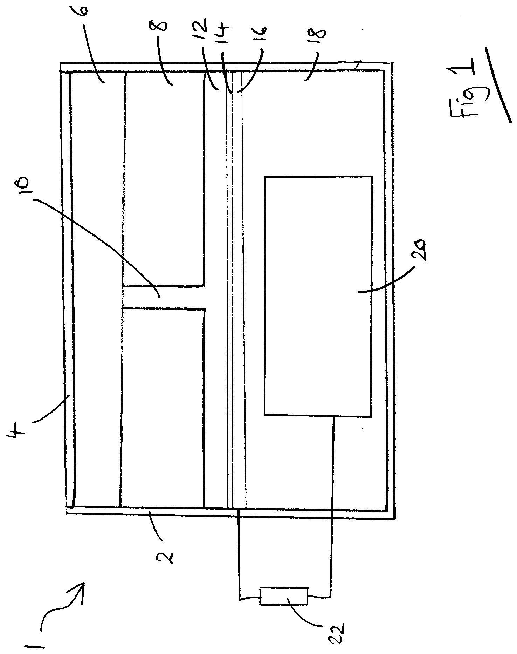

Figure 1 illustrates oxygen sensing apparatus shown generally as 1, within a flue gas analyser. The flue gas analyser also includes carbon monoxide sensing apparatus for measuring the concentration of carbon monoxide and optionally other gas sensing apparatus for measuring additional gases. The oxygen sensing apparatus includes a housing 2 and an inlet 4 defined by holes through which a gas sample can penetrate the housing. Crystals of potassium permanganate 6 (functioning as nitrogen-containing-gas removing means) are located in a chamber intermediate the inlet and a mass flow control member 8 which takes the form of a block of ABS through the middle of which a narrow (< 100 micron diameter) capillary tube 10 extends. The opposite end of the capillary tube opens into a gas space 12 bounded by the mass flow control member and a hydrophobic PTFE membrane 14, which functions as liquid permeable electrolyte retaining means. The gas space typically has a depth of only a few microns to minimise its volume. The hydrophobic PTFE membrane supports a catalyst 16 in the form of a cake of platinum, graphite and binder particles, which functions as the cathode. Gas permeates the catalyst cake through the hydrophobic PTFE membrane and hydrophobic binder particles. Electrolyte permeates the catalyst cake through the gaps between particles forming a three phase interface between analyte gas, electrolyte and catalyst within the cake.

The cathode is in gaseous communication with the gas space through the hydrophobic PTFE membrane and is in direct contact with electrolyte 18, based on potassium hydroxide, within an electrolyte chamber. A lead anode 20 is located within the electrolyte chamber, in direct contact with the electrolyte. A load resistor 22 completes a circuit between the anode and cathode. The electrolyte chamber, mass flow control member, gas space, hydrophobic PTFE membrane, cathode, anode, electrolyte and circuitry function as an electrochemical oxygen sensor.

In use, flue gas diffuses through the inlet into the potassium permanganate filled chamber. A range of gaseous species including nitrogen and oxygen, including nitrogen dioxide, is removed from the received gas by the potassium permanganate.

The substantial majority of the nitrogen dioxide is removed before the received gas reaches the capillary. The scrubbed gas diffuses through the capillary, gas space and PTFE membrane where oxygen is reduced by the cathode. Simultaneously, lead is oxidised to lead oxide at the anode and the resulting current is measured.

Essentially all of the oxygen which diffuses through the capillary is reduced and so, as with conventional mass transport limited electrochemical oxygen sensors, the output current depends on the rate of diffusion of oxygen through the capillary. In contrast to partial pressure electrochemical oxygen sensors, the output current depends on the oxygen concentration of gas received by the sensor rather than the partial pressure of oxygen outside the sensor.

We have found that, by providing nitrogen-containing-gas removing means for removing from received gas one or more gaseous species comprising nitrogen and oxygen which are either nitrogen dioxide, or formed from nitrogen dioxide in the presence of sufficient water vapour, which would otherwise lead to damage of the electrochemical oxygen sensor, the lifetime of the flue gas analyser is restored to normal, or near normal, when measuring the concentration of oxygen in flue gases which comprise nitrogen dioxide and which are saturated, or nearly saturated with water vapour.

### Experimental Results

Eight electrochemical gas sensors were used to measure oxygen in atmospheric air, with the addition of 1,000ppm nitrogen dioxide and water vapour to 90% relative humidity. All experiments were carried out at room temperature and pressure. Oxygen concentration was 20.9%. The electrochemical gas sensors used in all of the experiment were O2-A2 oxygen sensors from Alphasense Limited of Great Dunmow, UK, but similar results would be anticipated with comparable sensors from other suppliers. The oxygen sensor broadly corresponded to the apparatus described above and illustrated with reference to Figure 1, except that there was no nitrogen-containing-gas removing means. The sensors include a cathode comprising platinum, graphite and binder particles, a lead anode, a hydrophobic PTFE membrane covering and supporting the cathode and a mass flow control member comprising a capillary extending through a block of ABS.

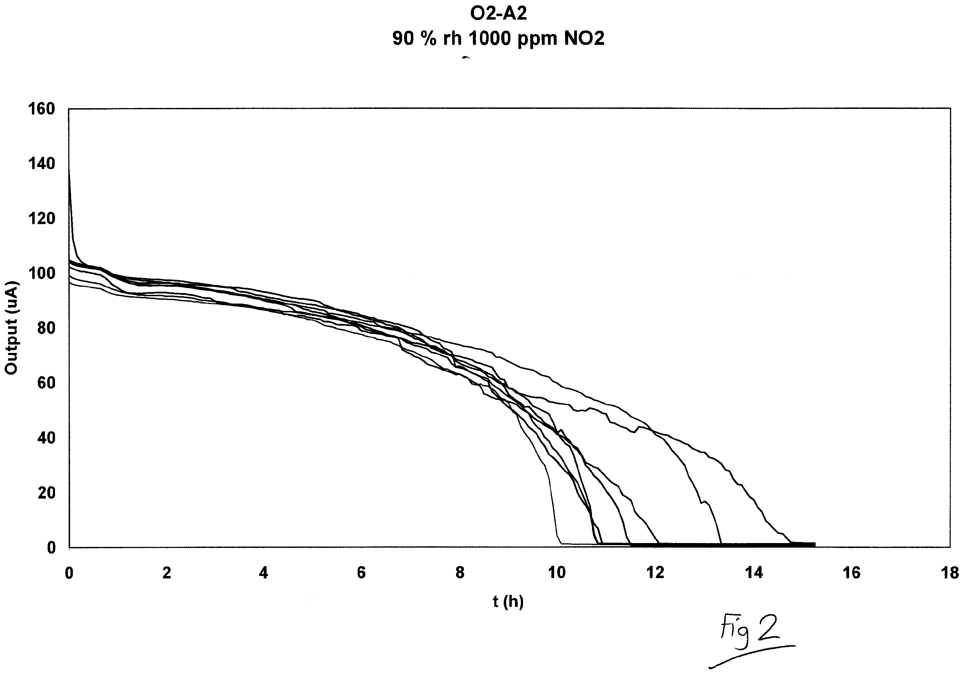

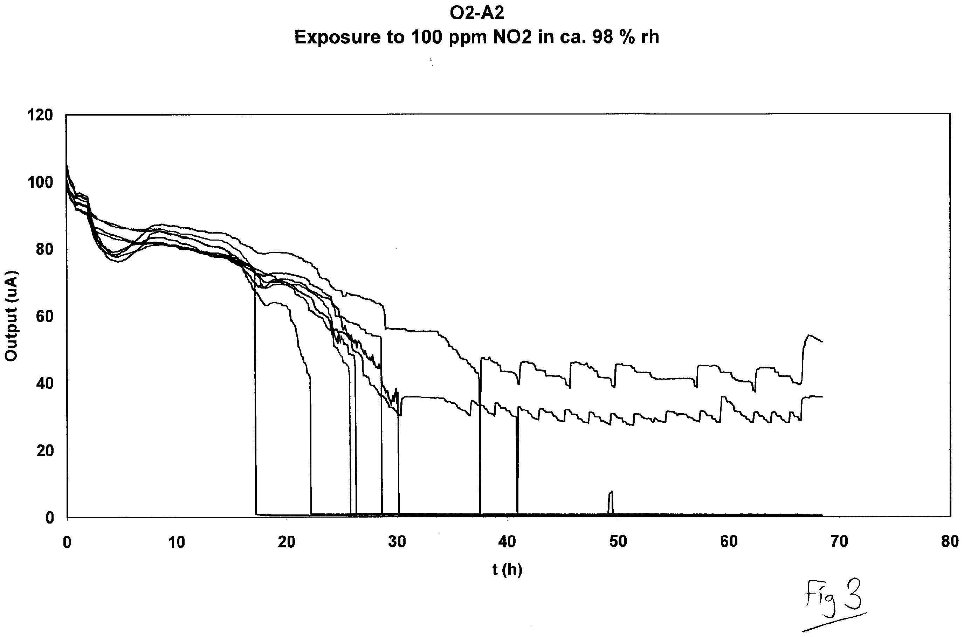

Figure 2 is a graph of the output current with time from the eight sensors. Although the O2-A2 sensors have a two year working life, the sensors failed catastrophically after 10 to 15 hours. Figure 3 is a graph of output current during an experiment with conditions which correspond to the previous experiments except that the nitrogen dioxide concentration was 100ppm and the relative humidity was 98%. In this case, 6 of the 8 sensors failed in 18 to 41 hours.

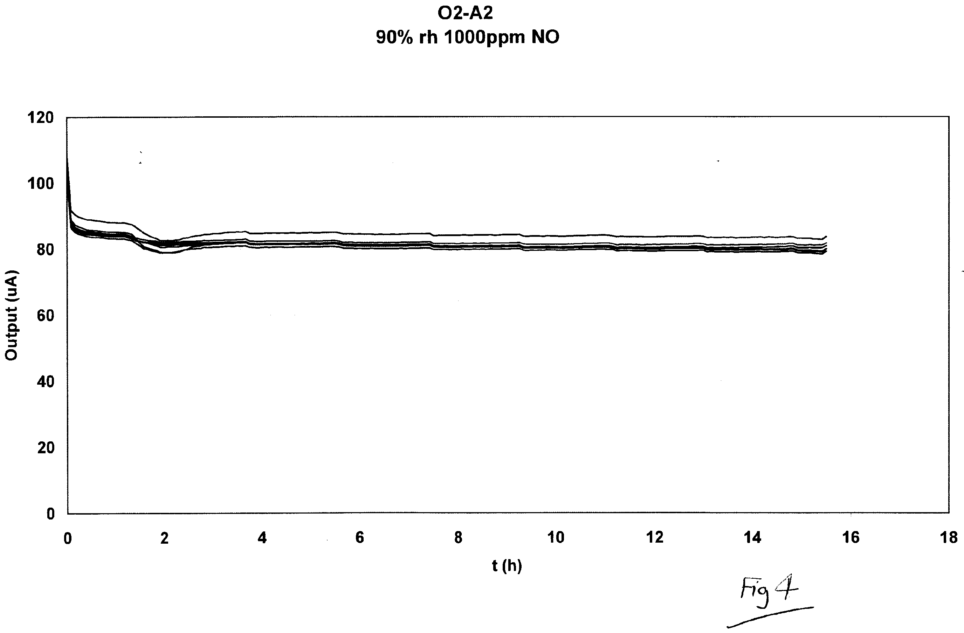

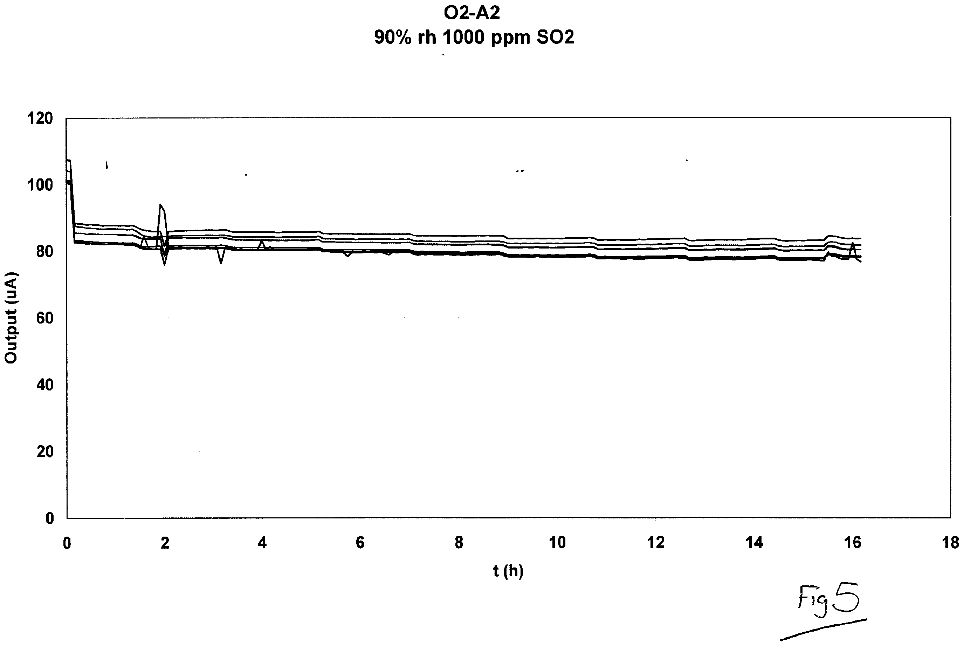

Corresponding experiments were conducted at 90% humidity and room temperature and pressure using 1,000ppm of nitric oxide or 1,000 sulfur dioxide instead of nitrogen dioxide. The results of these experiments, illustrated in Figures 4 and 5 respectively demonstrate that this effect arises in humid air specifically due to nitrogen dioxide. In these experiments, the decline in output current in the first minutes of the experiment is caused by dilution of oxygen by nitrogen from the gas bottles which supplied the nitric oxide and sulfur dioxide.

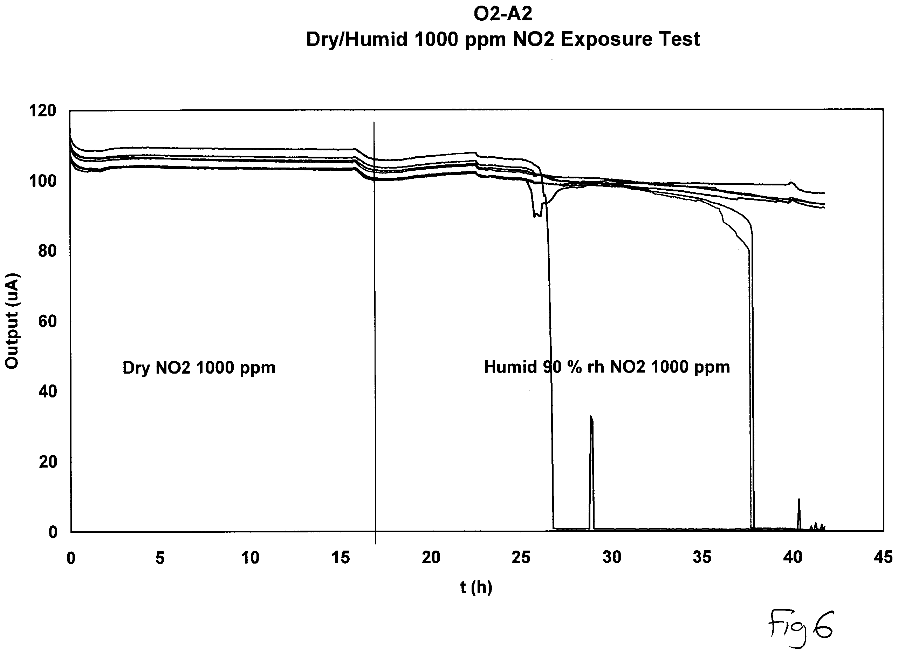

Figure 6 illustrates an experiment in which eight oxygen sensors were exposed to dry atmospheric air including 1,000ppm nitrogen dioxide for 17 hours, followed by atmospheric air including 1,000ppm nitrogen dioxide at a relative humidity of 90%, at room temperature and pressure throughout. The results of the experiment demonstrate that the sensors were not affected by 1,000ppm nitrogen dioxide, but that they were damaged in the presence of both nitrogen dioxide and a relatively high concentration of water vapour.

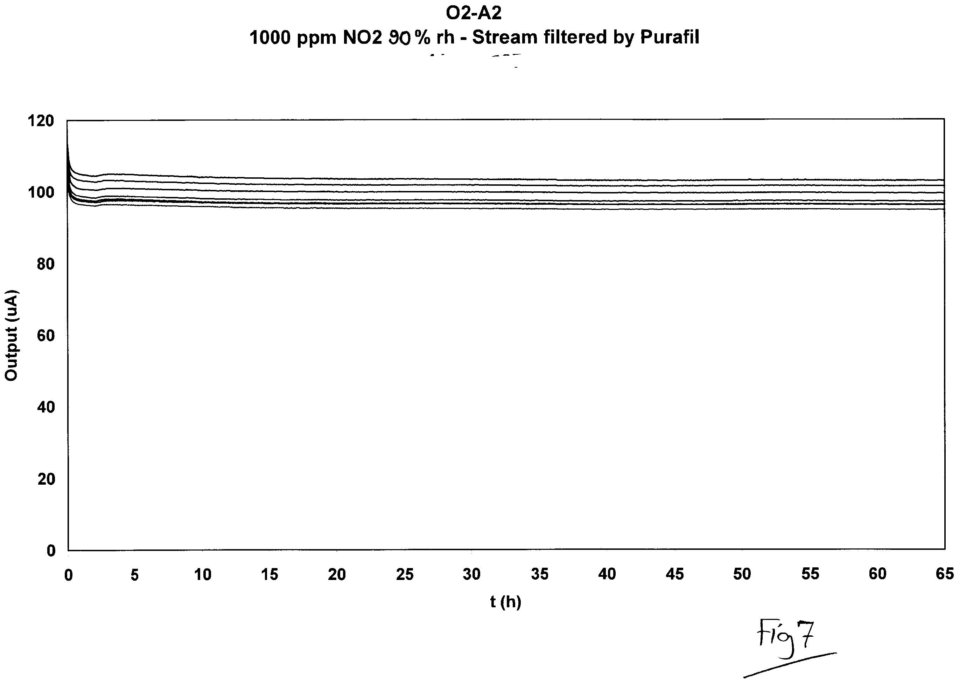

Figure 7 illustrate the results of an experiment in which the output current from eight oxygen sensors was monitored in the presence of gas comprising atmospheric air to which had been introduced 1,000 ppm nitrogen dioxide and water vapour to give a relative humidity of 95% which was continuously filtered through a Purafil brand filter from Purafil, Inc. of Doraville, USA. (Purafil is a trade mark). The Purafil brand filter includes potassium permanganate and is operable to chemisorb nitrogen dioxide, thereby functioning as the nitrogen-containing-gas removing means. As is apparent from Figure 7, the inclusion of the filter enabled the oxygen sensors to perform as normal.

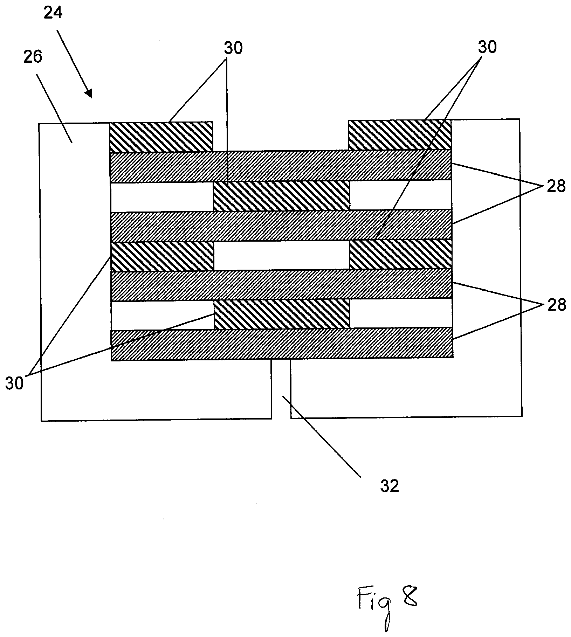

Figure 8 illustrates an alternative filter 24 comprising a filter housing 26 which replaces inlet 4 and potassium permanganate filled chamber 6 of the apparatus illustrated in Figure 1 to filter received gas before it reaches the capillary tube 10. The filter housing employs four sheets of activated carbon cloth 28 (type 150SL, available from Calgon Carbon Corporation of Pittsburgh, USA). The activated carbon cloth is arranged in layers spaced apart by intermediate gas-impermeable polymer spacers 30 which are configured to create a tortuous path for gas to diffuse through the sensor. For example, where the sensor is generally cylindrical, the gas-impermeable polymer members can be alternatively annular (having a central hole) and circular members which do not overlap significantly so that gas must diffuse alternately radially outwards and radially inwards in order to pass through the filter. The provision of a tortuous gas diffusion pathway maximises the surface area of carbon which is available to adsorb nitrogen dioxide from the received gas and increases the residence time of received gas within the filter. The configuration of the gas diffusion pathway and the internal surface area of the activated carbon cloth have been selected so that the filter adsorbs substantially all of the received nitrogen dioxide. A passage 32 forms an outlet to the filter housing which communicates with the capillary tube. The passage 32 is much broader than the capillary tube so that it is not diffusion limiting. For example, the passage may have a diameter of around 2mm.

### Discussion

We have demonstrated that electrochemical oxygen sensors are degraded in the presence of even a low concentration of nitrogen dioxide, in air which is saturated, or nearly saturated, with water vapour and demonstrated that this degradation can be prevented using nitrogen-containing-gas removing means (such as a gas removing filter) which removes from received gas one or more gaseous species including nitrogen and oxygen, which is either nitrogen dioxide or formed from nitrogen dioxide in the presence of sufficient water vapour, which would otherwise lead to damage of the electrochemical oxygen sensor.

A number of different nitric oxides (referred to generally as NOx) are found in flue gas, including nitrogen dioxide (NO₂), nitric oxide (NO), nitrous oxide (N₂O), the nitrate radical (NO₃), nitric acid (HNO₃), nitrous acid (HNO₂) and dinitrogen pentoxide (N₂O₅). Nitric oxides are formed by a number of different routes. Thermal NOx are formed at high temperatures (>1,600°C), where molecular oxygen and nitrogen disassociate into their atomic states. Fuel NOx results from the combustion of fossil fuels, such as coal, gas and oil, and biofuels, such as wood, where nitrogen contained within the fuel is released as a free radical which forms nitric oxide. Fuel NOx forms up to 50% of NOx when combusting oil and 80% of NOx when combusting coal.

In boilers, the majority of NOx is in the form of nitric oxide. However, there are a number of different mechanisms by which nitric oxide can be converted to nitrogen dioxide. Nitrogen dioxide is generated by the reaction between nitric oxide and certain hydrocarbons, such as ethylene, at low temperatures; nitrogen dioxide is generated by the reaction between nitric oxide and peroxy radicals or HO₂; nitrogen dioxide is rapidly generated by the reaction between nitric oxide and ozone. Nitrogen dioxide is also generated by the reaction between nitric oxide and oxygen. The rate of this reaction is generally slow, but it can be accelerated by water, particulates (giving heterogenous catalytic sites) and some others materials which could be found inside boilers.

We suggest a possible mechanism by which the presence of nitrogen dioxide could lead to electrochemical oxygen sensors being damaged only in very humid conditions. The nitrate radical is formed rapidly from the reaction between ozone and nitrogen dioxide. Ozone can be formed from the reaction between nitrogen dioxide and oxygen. Nitrogen dioxide and the nitrate radical react to form dinitrogen pentoxide, which is very soluble in water. In a humid environment, almost all nitrate radicals will be converted to dinitrogen pentoxide. Dinitrogen pentoxide is known to be converted to nitric acid when dissolved in water droplets. Accordingly, concentrated nitric acid may be formed from nitrogen dioxide in very humid conditions, for example the conditions found in condensing boilers. In this case, the provision of means to remove one or more of nitrogen dioxide, dinitrogen pentoxide or nitric acid gas could prevent damage to electrochemical oxygen sensors in which the mass flow control member or gas permeable liquid impermeable barrier is made from a material (typically a plastics material) which is damaged in the presence of nitrogen dioxide and a sufficient concentration of water vapour, perhaps due to the formation of nitric acid.

In very humid air containing nitrogen dioxide, mass flow control members made from plastics materials, such as ABS or polycarbonate, could be damaged. This damage could be prevented by providing the nitrogen-containing-gas removing means, upstream of the mass flow control member, or within the mass flow control member, for example within the capillary. In very humid air containing nitrogen dioxide, gas permeable liquid impermeable barriers made from plastics materials, such as PTFE, could be damaged, for example their surface energy might change enabling electrolyte to penetrate the barrier, causing the gas space to be flooded. This damage could be prevented by providing nitrogen-containing-gas removing means upstream of the gas permeable liquid impermeable barrier, for example, before the mass flow control member, within the mass flow control member, or within the gas space between the mass flow control member and the gas permeable liquid impermeable barrier.

Further modifications and variations may be made within the scope of the invention herein disclosed.

## Claims

1. Use of nitrogen-containing-gas removing means (6) to avoid failure of a mass flow controlled electrochemical oxygen sensor (1) in a flue gas analyser when measuring the concentration of oxygen in a flue gas having a relative humidity of at least 80% and a nitrogen dioxide concentration of at least 5ppm, the flue gas analyser comprising an inlet (4) for receiving gas for analysis from a flue, the mass flow controlled electrochemical oxygen sensor (1) comprising a cathode (16) which is operable to reduce oxygen and a mass flow control member (8) which is a narrow tube and which restricts the diffusion of flue gas to the cathode such that the reduction of oxygen at the cathode is mass flow limited, the nitrogen-containing-gas removing means removing from the received flue gas one or more gaseous species comprising nitrogen and oxygen which are either nitrogen dioxide, or formed from nitrogen dioxide in the presence of sufficient water vapour.

2. Use of nitrogen-containing-gas removing means (6) according to Claim 1, wherein the nitrogen-containing-gas removing means is nitric or nitrous acid removing means for removing nitric or nitrous acid from received gas.

3. Use of nitrogen-containing-gas removing means (6) according to Claim 1, wherein the nitrogen-containing-gas removing means is nitrogen dioxide removing means for removing nitrogen dioxide from received gas.

4. Use of nitrogen-containing-gas removing means (6) according to any preceding Claim, wherein the nitrogen-containing-gas removing means is operable to remove a range of acid-forming gases including nitrogen monoxide, nitrogen dioxide, dinitrogen pentoxide and sulfur dioxide from received gas.

5. Use of nitrogen-containing-gas removing means (6) according to any one of Claims 1 to 3, wherein the nitrogen-containing-gas removing means comprises a filter (24).

6. Use of nitrogen-containing-gas removing means (6) according to Claim 5, wherein the filter comprises a permanganate salt.

7. Use of nitrogen-containing-gas removing means (6) according to any one of Claims 1 to 6, wherein the nitrogen-containing-gas removing means changes visual properties and thereby provides a visual indicator when the nitrogen-containing-gas removing means is coming towards the end of its operational life.

8. Use of nitrogen-containing-gas removing means (6) according to Claim 1, wherein the nitrogen-containing-gas removing means is provided within the mass flow control member (8).

9. Use of nitrogen-containing-gas removing means (6) according to Claim 8, wherein the nitrogen-containing-gas removing means is provided intermediate the inlet (4) and the mass flow control member (8).

10. Use of nitrogen-containing-gas removing means (6) according to any one of Claims 8 to 9, the electrochemical oxygen sensor comprising a gas permeable liquid impermeable barrier (14) intermediate the cathode (16) and a gas space (12), the gas space being intermediate the gas permeable liquid impermeable barrier and the mass flow control member (8), wherein the nitrogen-containing-gas removing means is provided within the gas space.

11. Use of nitrogen-containing-gas removing means (6) according to any one of Claims 1 to 10, the flue gas analyser further comprising a nitrogen oxide sensor which is operable to measure nitrogen dioxide, wherein the nitrogen oxide sensor is located intermediate the inlet (4) and the nitrogen-containing-gas removing means or is in gaseous communication with the inlet other than through the nitrogen-containing-gas removing means.

## Patentansprüche

1. Verwendung eines Mittels (6) zum Entfernen von Stickstoff enthaltendem Gas für das Vermeiden eines Fehlers eines massenstromgesteuerten elektrochemischen Sauerstoffsensors (1) in einem Rauchgasanalysator beim Messen der Konzentration von Sauerstoff in einem Rauchgas mit einer relativen Luftfeuchtigkeit von mindestens 80 % und einer Stickstoffdioxid-Konzentration von mindestens 5 ppm, wobei der Rauchgasanalysator einen Einlass (4) zum Empfangen von Gas zur Analyse aus einem Rauch umfasst, wobei der massenstromgesteuerte elektrochemische Sauerstoffsensor (1) eine Kathode (16) umfasst, die zum Reduzieren von Sauerstoff betriebsfähig ist, und ein Massenstrom-Steuerelement (8), das ein enges Rohr ist und die Diffusion von Rauchgas zu der Kathode einschränkt, sodass die Reduktion von Sauerstoff an der Kathode massenstrombegrenzt ist, wobei das Mittel zum Entfernen von Stickstoff enthaltendem Gas aus dem empfangenen Rauchgas eine oder mehrere gasförmige Spezies entfernt, die Stickstoff und Sauerstoff umfassen und entweder Stickstoffdioxid oder in der Anwesenheit von ausreichend Wasserdampf aus Stickstoffdioxid gebildet sind.

2. Verwendung eines Mittels (6) zum Entfernen von Stickstoff enthaltendem Gas nach Anspruch 1, wobei das Mittel zum Entfernen von Stickstoff enthaltendem Gas ein Mittel zum Entfernen von Salpeter- oder salpetrige Säure für das Entfernen von Salpeter- oder salpetriger Säure aus empfangenem Gas ist.

3. Verwendung eines Mittels (6) zum Entfernen von Stickstoff enthaltendem Gas nach Anspruch 1, wobei das Mittel zum Entfernen von Stickstoff enthaltendem Gas ein Mittel zum Entfernen von Stickstoffdioxid für das Entfernen von Stickstoffdioxid aus empfangenem Gas ist.

4. Verwendung eines Mittels (6) zum Entfernen von Stickstoff enthaltendem Gas nach einem vorstehenden Anspruch, wobei das Mittel zum Entfernen von Stickstoff enthaltendem Gas für das Entfernen einer Reihe von säurebildenden Gasen aus empfangenem Gas betriebsfähig ist, darunter Stickstoffmonoxid, Stickstoffdioxid, Distickstoffpentoxid und Schwefeldioxid.

5. Verwendung eines Mittels (6) zum Entfernen von Stickstoff enthaltendem Gas nach einem der Ansprüche 1 bis 3, wobei das Mittel zum Entfernen von Stickstoff enthaltendem Gas ein Filter (24) umfasst.

6. Verwendung eines Mittels (6) zum Entfernen von Stickstoff enthaltendem Gas nach Anspruch 5, wobei das Filter ein Permanganatsalz umfasst.

7. Verwendung eines Mittels (6) zum Entfernen von Stickstoff enthaltendem Gas nach einem der Ansprüche 1 bis 6, wobei das Mittel zum Entfernen von Stickstoff enthaltendem Gas visuelle Eigenschaften ändert und dadurch einen visuellen Indikator bereitstellt, wenn das Mittel zum Entfernen von Stickstoff enthaltendem Gas zu dem Ende seiner Betriebslebensdauer kommt.

8. Verwendung eines Mittels (6) zum Entfernen von Stickstoff enthaltendem Gas nach Anspruch 1, wobei das Mittel zum Entfernen von Stickstoff enthaltendem Gas in dem Massenstrom-Steuerelement (8) vorgesehen ist.

9. Verwendung eines Mittels (6) zum Entfernen von Stickstoff enthaltendem Gas nach Anspruch 8, wobei das Mittel zum Entfernen von Stickstoff enthaltendem Gas zwischen dem Einlass (4) und dem Massenstrom-Steuerelement (8) vorgesehen ist.

10. Verwendung eines Mittels (6) zum Entfernen von Stickstoff enthaltendem Gas nach einem der Ansprüche 8 bis 9, wobei der elektrochemische Sauerstoffsensor eine gasdurchlässige flüssigkeitsundurchlässige Barriere (14) zwischen der Kathode (16) und einem Gasraum (12) umfasst, wobei sich der Gasraum zwischen der gasdurchlässigen flüssigkeitsundurchlässigen Barriere und dem Massenstrom-Steuerelement (8) befindet, wobei das Mittel zum Entfernen von Stickstoff enthaltendem Gas in dem Gasraum vorgesehen ist.

11. Verwendung eines Mittels (6) zum Entfernen von Stickstoff enthaltendem Gas nach einem der Ansprüche 1 bis 10, wobei der Rauchgasanalysator weiter einen Stickstoffoxidsensor umfasst, der zum Messen von Stickstoffdioxid betriebsfähig ist, wobei sich der Stickstoffoxidsensor zwischen dem Einlass (4) und dem Mittel zum Entfernen von Stickstoff enthaltendem Gas befindet oder mit dem Einlass in einer anderen Gasverbindung steht als durch das Mittel zum Entfernen von Stickstoff enthaltendem Gas.

## Revendications

1. Utilisation de moyen d'élimination de gaz contenant de l'azote (6) pour éviter une défaillance d'un capteur électrochimique d'oxygène à flux de masse contrôlé (1) dans un analyseur de gaz de combustion lors d'une mesure de la concentration d'oxygène dans un gaz de combustion ayant une humidité relative d'au moins 80 % et une concentration de dioxyde d'azote d'au moins 5 ppm, l'analyseur de gaz de combustion comprenant une entrée (4) pour recevoir du gaz à analyser à partir d'un conduit de fumée, le capteur électrochimique d'oxygène à flux de masse contrôlé (1) comprenant une cathode (16) qui est apte à réduire de l'oxygène et un élément de contrôle de flux de masse (8) qui est un tube étroit et qui restreint la diffusion de gaz de combustion vers la cathode de telle sorte que la réduction d'oxygène au niveau de la cathode a un flux de masse limité, le moyen d'élimination de gaz contenant de l'azote éliminant, du gaz de combustion reçu, une ou plusieurs espèces gazeuses comprenant de l'azote et de l'oxygène qui sont soit du dioxyde d'azote, soit formées à partir de dioxyde d'azote en présence de vapeur d'eau suffisante.

2. Utilisation de moyen d'élimination de gaz contenant de l'azote (6) selon la revendication 1, dans laquelle le moyen d'élimination de gaz contenant de l'azote est un moyen d'élimination d'acide nitrique ou nitreux pour éliminer de l'acide nitrique ou nitreux à partir du gaz reçu.

3. Utilisation de moyen d'élimination de gaz contenant de l'azote (6) selon la revendication 1, dans laquelle le moyen d'élimination de gaz contenant de l'azote est un moyen d'élimination de dioxyde d'azote pour éliminer du dioxyde d'azote à partir du gaz reçu.

4. Utilisation de moyen d'élimination de gaz contenant de l'azote (6) selon l'une quelconque des revendications précédentes, dans laquelle le moyen d'élimination de gaz contenant de l'azote est apte à éliminer une plage de gaz de formation d'acide comprenant du monoxyde d'azote, du dioxyde d'azote, du pentoxyde d'azote et du dioxyde de soufre, à partir du gaz reçu.

5. Utilisation de moyen d'élimination de gaz contenant de l'azote (6) selon l'une quelconque des revendications 1 à 3, dans laquelle le moyen d'élimination de gaz contenant de l'azote comprend un filtre (24).

6. Utilisation de moyen d'élimination de gaz contenant de l'azote (6) selon la revendication 5, dans laquelle le filtre comprend un sel de permanganate.

7. Utilisation de moyen d'élimination de gaz contenant de l'azote (6) selon l'une quelconque des revendications 1 à 6, dans laquelle le moyen d'élimination de gaz contenant de l'azote change des propriétés visuelles et, de ce fait, fournit un indicateur visuel lorsque le moyen d'élimination de gaz contenant de l'azote se rapproche de sa fin de vie utile.

8. Utilisation de moyen d'élimination de gaz contenant de l'azote (6) selon la revendication 1, dans laquelle le moyen d'élimination de gaz contenant de l'azote est disposé à l'intérieur de l'élément de contrôle de flux de masse (8).

9. Utilisation de moyen d'élimination de gaz contenant de l'azote (6) selon la revendication 8, dans laquelle le moyen d'élimination de gaz contenant de l'azote est disposé entre l'entrée (4) et l'élément de contrôle de flux de masse (8).

10. Utilisation de moyen d'élimination de gaz contenant de l'azote (6) selon l'une quelconque des revendications 8 et 9, dans laquelle le capteur électrochimique d'oxygène comprend une barrière perméable aux gaz et imperméable aux liquides (14) entre la cathode (16) et un espace de gaz (12), l'espace de gaz étant entre la barrière perméable aux gaz et imperméable aux liquides et l'élément de contrôle de flux de masse (8), le moyen d'élimination de gaz contenant de l'azote étant disposé à l'intérieur de l'espace de gaz.

11. Utilisation de moyen d'élimination de gaz contenant de l'azote (6) selon l'une quelconque des revendications 1 à 10, dans laquelle l'analyseur de gaz de combustion comprend en outre un capteur d'oxyde d'azote qui est apte à mesurer du dioxyde d'azote, le capteur d'oxyde d'azote étant situé entre l'entrée (4) et le moyen d'élimination de gaz contenant de l'azote ou est en communication gazeuse avec l'entrée autrement que par l'intermédiaire du moyen d'élimination de gaz contenant de l'azote.
